# FASCICULE DE BREVET EUROPEEN

(11) **EP 0 886 480 B1**
(45) Date de publication et mention de la délivrance du brevet: **12.12.2001**
(21) Numéro de dépôt: 97914409.4
(22) Date de dépôt: 14.03.1997
(51) Int. Cl.: A41D 27/24, A41F 9/02

(54) **COULISSE DE FRONCAGE ET SON PROCEDE DE FABRICATION**
FALTENHÜLSE UND VERFAHREN ZU IHRER HERSTELLUNG
RUFFLING SLIDE AND METHOD FOR MAKING SAME

(30) Priorité: 15.03.1996 FR 9603548
(43) Date de publication de la demande: 30.12.1998
(73) Titulaire: CERA FRANCE COMPAGNIE D'EQUIPEMENT ROBOTIQUE APPLIQUEE, 42390 Villars (FR)
(72) Inventeur: COMBE, Robert, F-42450 Sury-le-Comtal (FR); MOULIN, Serge, F-42450 Sury-le-Comtal (FR)
(74) Mandataire: Ropital-Bonvarlet, Claude
(86) Numéro de dépôt international: FR9700470
(87) Numéro de publication internationale: WO9734506

(56) Documents cités:
- EP-A- 0 300 615
- EP-A- 0 677 284
- WO-A-96/01180
- FR-A- 2 532 337
- US-A- 4 333 978

## Description

### DOMAINE TECHNIQUE :

La présente invention est relative au domaine général des articles réalisés à base de nappes fibreuses et pour lesquels il est utile ou nécessaire de pouvoir établir, par fronçage préalable, une zone locale fonctionnelle d'étirement élastique possible limité.

Par nappes fibreuses, il convient de considérer tous les supports ou substrats à base de fibres naturelles et/ou synthétiques organisées de façon structurée comme par obtention par tissage ou organisées de façon aléatoire comme les supports non tissés ou les nappes ou voiles cardés, voire légèrement aiguilletés. Des articles répondant à de telles caractéristiques sont utilisés dans de très nombreux domaines dont, à titre d'exemples et pour faciliter la compréhension, il est simplement cité celui de l'habillement ainsi que celui des articles d'hygiène, notamment, pour les enfants en bas âge et par exemple les couches-culottes.

### TECHNIQUE ANTÉRIEURE :

Dans les articles ci-dessus, il est généralement souhaité pouvoir disposer d'une ou plusieurs zones de fronçage de manière que la zone en question puisse trouver une faculté d'adaptation, de conformation ou de rétention compatible avec le ou les objectifs visés.

Jusqu'à présent, on a voulu répondre à de telles exigences en ayant recours à différentes méthodes faisant intervenir à chaque fois un ou plusieurs éléments filaires étirables longitudinalement.

L'un des procédés connus consiste à lier l'élément filaire étiré longitudinalement avec le substrat au moyen d'un cordon ou de points de collage d'un produit adhésif intermédiaire.

Une telle technique pose de gros problèmes de mise en service, notamment dans les installations de fabrication en continu, car il est nécessaire de pouvoir disposer d'une régulation en température particulièrement précise pour que le dépôt du cordon ou des points de produit adhésif puisse intervenir avec précision locale, sans débordement, sans filage, sans colmatage de l'installation de distribution et avec la quantité déposée juste nécessaire, toutes ces exigences et conditions devant pouvoir être maintenues de façon constante pour des vitesses de défilement de fabrication des articles relativement grandes.

On conçoit qu'une telle régulation de température pour des dépôts de faible quantité pose un problème quasiment insoluble pour la technique actuelle.

Un autre inconvénient de cette technique réside dans le fait que le produit adhésif est peu ou prou sensible à la poussière qui, par sa présence peut être de nature à perturber la liaison par collage entre le fil extensible et le substrat.

Un autre inconvénient de cette technique réside dans le fait que le produit adhésif peut connaître une migration désagréable à travers le substrat, notamment dans le cas de contre-collage, en conférant à l'article réalisé un aspect inesthétique, voire agressif lorsque le substrat doit être placé en relation directe avec l'épiderme. Une telle migration peut également être la source d'un contre-collage préjudiciable à la fonction locale devant être assumée. Tel est le cas des barrières fécales des couches-culottes dont l'efficacité est ruinée, si ces barrières ne possèdent plus, par collage sur le support, la liberté nécessaire pour être relevées sensiblement perpendiculairement au plan du support par l'action de fronçage du fil étirable.

Une autre technique de l'art antérieur consiste à lier le fil et le support au moyen d'une couture réalisée de manière à traverser localement le fil ou à emprisonner ce dernier sur le substrat.

Cette technique n'est pas satisfaisante pour différentes raisons.

Le coût de réalisation des zones froncées devient, dans certains cas et notamment pour les articles de grande consommation et d'usage unique, rédhibitoire en raison de la faible vitesse de production, de la dépense générée par la consommation du fil de liaison et par les interruptions fréquentes dues aux ruptures du fil, à la nécessité de rechargement des bobines distributrices de fils, voire aux changements des têtes de cousage en raison des ruptures ou cassures des aiguilles.

Un inconvénient particulièrement rédhibitoire de cette technologie réside aussi dans la conséquence résultant du travail des aiguilles qui sont amenées à traverser et donc à perforer le ou les substrats devant être garnis.

De telles perforations représentent des obstacles insurmontables et rédhibitoires dans le cas où il convient de réaliser des articles étanches, comme cela est le cas pour certains domaines scientifiques ou encore pour le domaine médical.

Afin de remédier aux inconvénients des techniques précitées, la demande de brevet FR 2 532 337 propose une autre technique qui consiste à mettre en oeuvre une coulisse de fronçage constituée d'une gaine tubulaire définie par le substrat et, par exemple, le repli d'un de ses bords. A l'intérieur de cette gaine, entre les deux nappes, est disposé le fil extensible qui est lié aux parois de la gaine par des zones de liaison transversale espacées, délimitant entre elles des sections tubulaires de fronçage à l'intérieur desquelles l'élément filaire est libre. Les zones de liaison assurent, également, l'assemblage entre elles des deux nappes de la gaine.

Une telle coulisse de fronçage est réalisée en continu, au moyen d'une installation disposant la gaine mise à plat, à l'état étendu et plaçant, dans cette dernière, l'élément filaire soumis à une contrainte d'étirement longitudinal. L'installation forme alors, par thermosoudage ou soudure à haute fréquence, localement et de manière alignée, les immobilisations locales de l'élément filaire sur et entre les deux nappes de la gaine. Après exécution de cette immobilisation, la contrainte sur l'élément filaire est relâchée, de sorte qu'il présente une longueur inférieure à celle de la section tubulaire comprise entre deux immobilisations et qu'il lui impose, par suite, un froncis d'orientation sensiblement perpendiculaire à la direction générale de la gaine.

Cette dernière technique permet, effectivement, d'apporter une solution aux problèmes soulevés par les autres techniques connues, mais possède, néanmoins, certains inconvénients résultant de la nature même des zones de liaison mises en oeuvre.

En effet, selon la demande FR 2 532 337, les zones de liaison, qui présentent une forme sensiblement rectangulaire, assurent, simultanément, un soudage entre les deux nappes constitutives de la gaine et une adhésion du fil extensible sur chacune de ses deux nappes, de sorte qu'il n'est pas possible d'assurer un réglage du fronçage après fabrication de la coulisse. De plus, les zones de liaison altèrent le comportement du fil extensible en engendrant des discontinuités dans sa structure. Ces discontinuités résultent, notamment, des contraintes mécaniques et thermiques dues à l'opération de soudage du fil sur les parois de la gaine. De même, l'alternance de sections tubulaires dans lesquelles le fil est libre et de zones de liaison au niveau desquelles le fil adhère aux parois de la gaine, ne permet pas de tirer le meilleur parti possible des caractéristiques mécaniques du fil qui se trouve, en quelque sorte, divisé en une succession d'éléments élastiques indépendants n'agissant qu'au niveau d'une seule section tubulaire pour froncer cette dernière.

### EXPOSÉ DE L'INVENTION :

L'objet de l'invention est justement de perfectionner la technique ci-dessus en proposant une nouvelle technologie capable de surmonter les inconvénients précités, qui permette de réaliser une coulisse de fronçage étanche, à faible prix, de grande capacité d'étirement pouvant être produite à l'unité ou en continu. L'objet de l'invention est aussi de proposer une technique permettant d'obtenir un produit nouveau n'exigeant lors de sa mise en oeuvre aucune disposition particulière de reprise au droit des coupes réalisées, en vue d'arrêter par liaison ponctuelle localisée l'élément filaire extensible longitudinalement par rapport à la coulisse. L'objet de l'invention est également de proposer une nouvelle technique qui permet d'obtenir une coulisse de fronçage sans altération des qualités mécaniques de l'élément filaire extensible.

L'objet de l'invention est de proposer une nouvelle technologie de coulisse de fronçage qui offre des capacités d'étirement ou de réactivité adaptées à l'application visée, qui puisse être produite à un faible coût et à très grande vitesse lorsque le procédé mis en oeuvre est celui de réalisation en continu et qui puisse faire l'objet d'un réglage du fronçage après fabrication.

Pour atteindre les objectifs ci-dessus, la coulisse de fronçage comprend :
- une gaine tubulaire destinée à contenir au moins un élément filaire étirable élastiquement,
- des zones de liaison qui sont aménagées transversalement dans la gaine en étant espacées pour délimiter entre elles des sections tubulaires de fronçage et qui réalisent des immobilisations locales dudit élément filaire,
- au moins un élément filaire disposé dans la gaine pour être retenu au droit des immobilisations et pour traverser librement et successivement les sections tubulaires en présentant localement dans chacune d'elles, d'une part, son état de repos et, d'autre part, une longueur inférieure à celle de la section à laquelle il impose, par suite, un froncis d'orientation sensiblement perpendiculaire à la direction générale de la gaine.

Selon l'invention, cette coulisse de fronçage est caractérisée en ce que chaque zone de liaison délimite un étranglement présentant une section de passage sensiblement voisine de celle à l'état étiré de l'élément filaire pour d'une part, autoriser un déplacement relatif de l'élément filaire sous l'effet d'une contrainte dite de réglage et, d'autre part, immobiliser l'élément filaire lorsque ce dernier est au repos ou soumis à une contrainte inférieure à la contrainte de réglage.

L'invention a aussi pour objet l'article nouveau comportant, de façon intégrée ou rapportée, une coulisse de fronçage conforme à l'invention.

L'invention a aussi pour objet un procédé pour l'obtention de la coulisse ci-dessus, un tel procédé consistant à :
- disposer d'une gaine mise à plat à l'état étendu,
- placer dans la gaine au moins un élément filaire étirable élastiquement qui est immobilisé localement et qui est soumis par ailleurs à une contrainte d'étirement longitudinal,
- réaliser localement dans la gaine, de façon espacée, des zones de liaison :
   . qui délimitent dans la gaine des sections tubulaires de fronçage,
   . et qui réalisent chacune une immobilisation constituée par un étranglement présentant une section de passage sensiblement voisine de celle de l'état étiré de l'élément filaire et retenant ce dernier pour, d'une part, autoriser un déplacement relatif de l'élément filaire sous l'effet d'une contrainte dite de réglage et, d'autre part, immobiliser l'élément filaire lorsque ce dernier est au repos ou soumis à une contrainte inférieure à la contrainte de réglage,
- et à relâcher la contrainte d'étirement appliquée à l'élément filaire qui se rétracte à l'état de repos entre les immobilisations et à l'intérieur de chaque section tubulaire à laquelle il impose un froncis.

Diverses autres caractéristiques ressortent de la description faite ci-dessous en référence aux dessins annexés qui montrent, à titre d'exemples non limitatifs, des formes de réalisation de l'objet de l'invention.

La **fig. 1** est une perspective partielle illustrant la coulisse de fronçage conforme à l'invention.

La **fig. 2** est une perspective partielle montrant, sous un autre angle, une autre caractéristique de l'objet de l'invention.

La **fig. 3** est une coupe partielle prise sensiblement selon un plan **III-III** de la **fig. 1.**

La **fig. 4** est une perspective schématique mettant en évidence une possibilité de réalisation de l'objet de l'invention.

Les **fig. 5** et **6** sont des vues schématiques montrant différentes variantes de réalisation de l'invention.

La **fig. 7** est une perspective schématique d'une installation pour la réalisation de la coulisse selon la **fig. 1.**

### MEILLEURE MANIÈRE DE RÉALISER L'INVENTION :

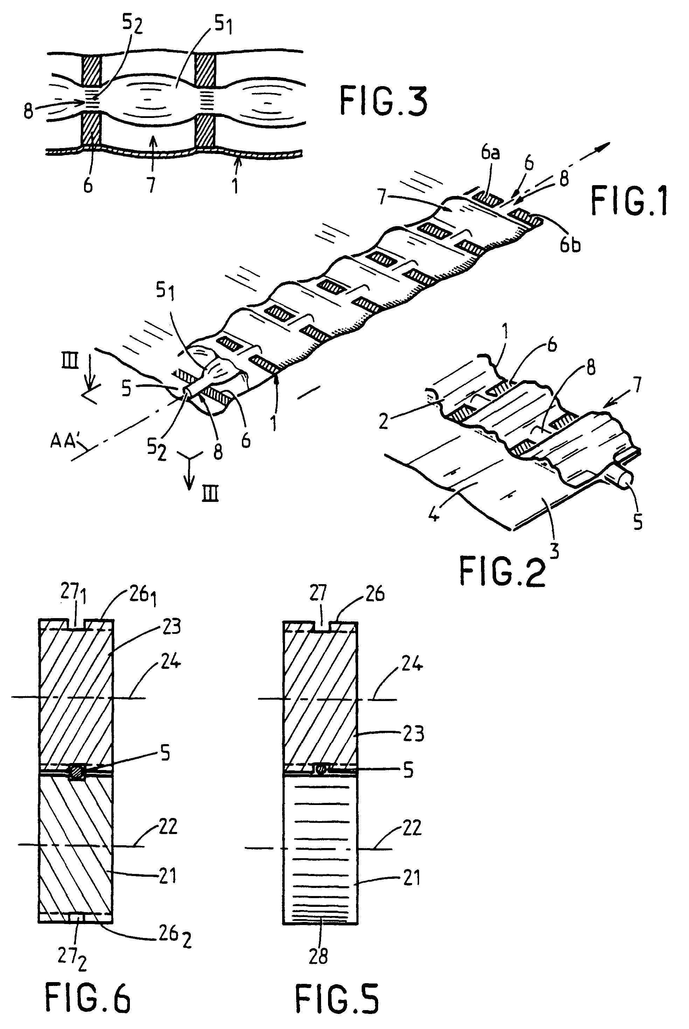

La coulisse de fronçage selon les **fig. 1** à **3** est constituée à partir d'un substrat fibreux de toute nature appropriée qui peut être tissé ou non tissé, voire dans certains cas être constitué par une feuille ou un film continu de matière plastique.

Dans le cas de substrats fibreux, toutes les fibres ou tous les filés de fibres convenables peuvent être retenus et plus particulièrement les fibres qui revêtent par elles-mêmes, voire par apport individuel, une faculté de thermosoudage.

Il peut, bien entendu, être envisagé de recourir à des substrats de fibres ne répondant pas à cette caractéristique de façon intrinsèque et, dans un tel cas, un dépôt préalable par enduction, pulvérisation, trempage d'un film de matière thermosoudable peut alors être réalisé sur le substrat.

La coulisse de fronçage comprend, principalement, une gaine tubulaire **1** qui peut être considérée comme étant réalisée antérieurement en tant que telle pour constituer un élément unitaire ou, encore, être formée in situ, en fonction des besoins, comme cela est illustré par la **fig. 2.** Dans un tel cas, la gaine est alors formée par un rempli ou repli **2** d'une bande **3** qui peut, le cas échéant et dans certaines applications, être constituée par la nappe devant présenter localement un caractère de fronçage extensible. Dans un tel cas, le rempli ou repli **2** est alors formé par une sorte d'ourlet ou de bordure établi en lisière de la nappe.

La **fig. 2** met aussi en évidence que le repli ou rempli **2** peut être formé à partir d'une bande pour n'intéresser qu'une partie de la largeur de cette dernière, de manière à laisser subsister une sorte de dépassant **4** permettant, le cas échéant, une liaison par couture, collage ou thermosoudage avec une nappe sur laquelle le dépassant doit être rapporté pour adapter la coulisse sur ladite nappe.

La gaine **1** est destinée à contenir au moins un élément filaire **5** étirable élastiquement de façon longitudinale et par exemple constitué par un fil mono ou pluri brins, éventuellement guipé.

A titre d'exemple et dans l'application aux produits d'hygiène tels que les couches-culottes, voire les charlottes, l'élément filaire **5** peut avantageusement être constitué par un fil de matière vendue dans le commerce sous la marque LYCRA® .

L'élément filaire **5** est destiné à être disposé à l'intérieur de la gaine **1** qui présente des zones de liaison **6** qui sont aménagées transversalement ou de façon inclinée par rapport à l'axe longitudinal **A-A'** de ladite gaine. Les zones **6** sont exécutées en étant espacées de manière équidistante ou non, de façon à délimiter entre elles et dans la gaine des sections tubulaires élémentaires successives de fronçage **7.** Dans l'exemple illustré, les zones de liaison **6** sont, par ailleurs, exécutées de manière que par chacune d'elles s'établisse localement une immobilisation de l'élément filaire **5.** Dans le cas présent, cette immobilisation résulte du fait que chaque zone de liaison **6** est exécutée pour que la section utile de la gaine **1** soit limitée à un étranglement **8** dont le passage est sensiblement voisin de la section droite transversale de l'élément filaire **5** à l'état étiré totalement ou partiellement. A cette fin, il peut être considéré que les zones **6** sont constituées chacune, par exemple, par deux pavés **6a** et **6b** qui sont aménagés de part et d'autre de l'axe longitudinal **A-A',** de manière à laisser subsister un étranglement **8.** Il doit être considéré que selon l'exemple des fig. **1** et **2**, l'axe **A-A'** est placé de façon médiane par rapport à la largeur de la gaine **1,** mais qu'une position non médiane pourrait aussi être envisagée. De même, la gaine **1** pourrait posséder une largeur non constante.

Les zones de liaison **6** sont exécutées par tout moyen approprié de manière à lier les deux épaisseurs constitutives de la gaine **1** mise à plat et étalée et par exemple un procédé faisant intervenir un thermosoudage peut être considéré comme une solution offrant de grands avantages.

Il pourrait être retenu de réaliser les zones de liaison **6** de manière que l'étranglement **8** soit situé, non pas en alignement avec l'axe **A-A'** considéré comme médian transversalement parlant, mais par exemple en relation avec l'une des bordures de la gaine correspondant à l'un des plis de sa structure mise à plat ou l'un des plis formé par le rempli **2.**

De préférence, mais non exclusivement, les zones de liaison **6** sont exécutées pour que les étranglements **8** soient alignés de manière rectiligne.

La coulisse de fronçage ci-dessus comprend par ailleurs un élément filaire **5** qui est disposé à l'intérieur de la gaine **1,** de manière à être retenu au droit des immobilisations **8** et à traverser librement les sections **7** successivement en présentant, toutefois, son état de repos tel que **5**_{**1**} à l'intérieur de chaque section tubulaire.

Une disposition constructive supplémentaire fait intervenir une mesure axiale de l'élément filaire **5,** comprise entre deux immobilisations **8,** inférieure à l'état de repos à la longueur élémentaire de chaque section **7.**

En raison de l'ancrage relatif qui est établi au droit de chaque immobilisation **8** et en raison de cette différence de longueur, la coulisse de fronçage présente, à l'état de repos, un froncis localisé au droit de chaque section tubulaire **7,** comme cela ressort de la **fig. 2.** Dans cet état de repos, il n'existe aucune possibilité de déplacer relativement l'élément filaire **5** qui est immobilisé axialement par les immobilisations **8** emprisonnant par les étranglements les zones **5**_{**2**} successives de l'élément filaire **5.**

Il est, toutefois, possible d'obtenir, lorsqu'un réglage du fronçage de la coulisse est souhaité, un déplacement relatif axial de l'élément filaire **5** par rapport aux immobilisations **8.** A cette fin, il convient d'exercer, sur l'élément filaire, une contrainte ou traction axiale, dite de réglage, telle que les dimensions de la section droite transversale de l'élément filaire **5** soient inférieures à celles des étranglements **8.**

Lorsque la gaine ci-dessus est soumise à une contrainte d'étirement inférieure à la contrainte de réglage, les zones **5**_{**1**} de l'élément filaire sont étirées et permettent l'extension de chaque section **7** sans, toutefois, provoquer de déplacement relatif de l'élément filaire **5** sur l'axe **A-A',** étant donné l'emprisonnement relatif au droit de chaque immobilisation par la zone étirée **5**_{**2**}**.** Lorsque la contrainte d'étirement élastique cesse, la coulisse de fronçage est sollicitée par les différentes zones **5**_{**1**} qui ont tendance à reprendre leur état de repos et à réimposer le froncis local de chaque section **7.** De plus, en raison de l'absence de points d'adhérence entre la gaine **1** et l'élément filaire **5,** lorsque la coulisse est soumise à une contrainte d'étirement, toute la longueur de l'élément filaire est mise à contribution pour s'opposer à l'action de cette contrainte, contrairement à ce qui se passerait dans le cas d'une coulisse de fronçage selon l'art antérieur.

La coulisse de fronçage, du type décrit ci-dessus, présente un avantage particulièrement intéressant dans la mise en oeuvre lorsqu'elle constitue plus particulièrement, mais non exclusivement, un élément tubulaire indépendant. En effet, en raison de la présence des zones **5**_{**1**} qui, à l'état de repos, présentent obligatoirement une section supérieure à celle des immobilisations **8,** une coupe transversale à longueur voulue de la gaine peut intervenir sans aucun égard, ni aucune opération de reprise. En effet chaque zone **5**_{**1**} extrême et coupée représente, par la section qu'elle présente à l'état de repos, une butée d'arrêt maintenant la relation de tension existant entre l'élément filaire **5** et la gaine **1** par l'intermédiaire des immobilisations **8** et des sections **7** successives.

Il doit, bien entendu, être considéré que la coulisse de fronçage pourrait comporter plusieurs alignements d'immobilisation **8,** réservés à autant d'éléments filaires **5** et que, dans certains cas, il pourrait aussi être envisagé de réaliser, pour chaque alignement, des immobilisations **8** qui ne seraient pas exactement établies dans un alignement rectiligne, mais pourraient définir, pour un élément filaire **5,** un trajet incluant des inflexions plus ou moins prononcées, voire des courbes alternées.

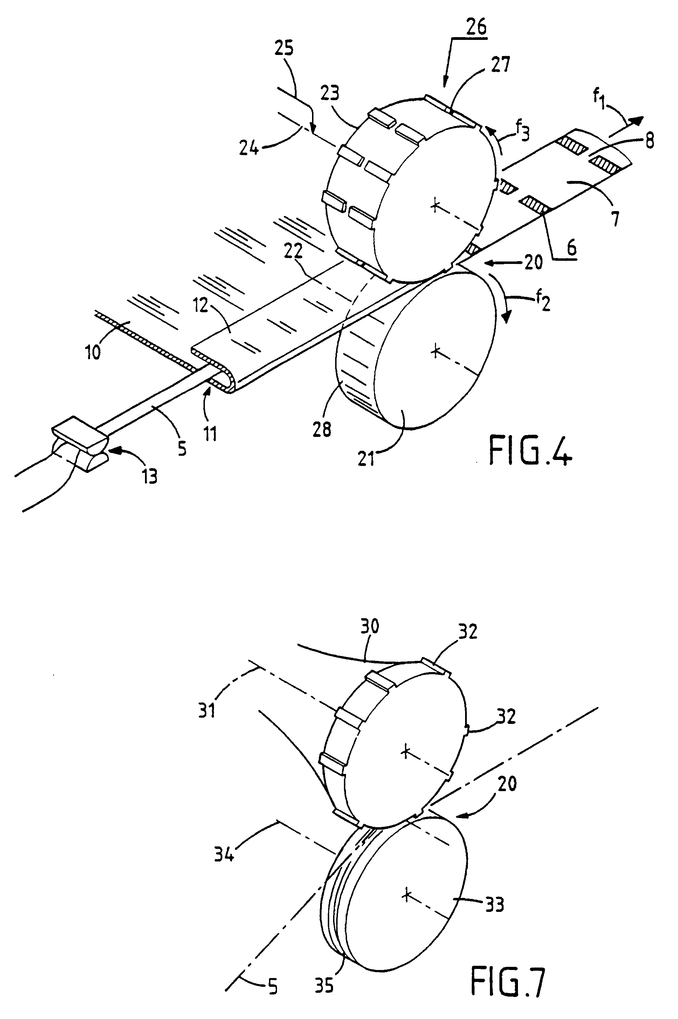

La **fig. 4** illustre de façon schématique une installation pour la mise en oeuvre d'un procédé de fabrication de la coulisse de fronçage selon les **fig. 1** à **3.** Un tel procédé correspond à une réalisation faisant intervenir une nappe **10** à partir de laquelle une gaine **11** est formée par un rempli de bordure **12** destiné à constituer, directement sur la nappe **10,** la coulisse de fronçage.

Les moyens mis en oeuvre pour assurer le repli ou le rempli de bordure **12** n'entrent pas directement dans le cadre de l'invention, car ils doivent être considérés comme directement accessibles à l'homme de métier confronté à l'exécution, par exemple en matière textile, d'un ourlet en continu.

La formation de la gaine **11** prend en compte l'insertion d'un élément filaire **5** qui est maintenu entre les couches constitutives du pli ou de la gaine en étant soumis à une contrainte d'extension élastique dans le sens de la flèche **f**_{**1**}**,** par exemple à partir d'un dispositif de freinage **13,** tel qu'un guichet ou des mâchoires de pince limitant son défilement libre, de manière à produire une mise sous tension en amont d'un dispositif **20** de formation de la coulisse.

La gaine **11** et l'élément filaire **5** sont astreints à défiler en relation avec le dispositif **20** qui comprend un duo de molettes dont l'une, par exemple **21,** est dite embase ou enclume et qui est entraînée en rotation sur son axe **22** de manière passive ou active dans le sens de la flèche **f**_{**2**}**.** Le duo **20** comprend une autre molette **23,** dite de formage, entraînée en rotation dans le sens de la flèche **f**_{**3**} sur son axe **24** qui est relié par tout moyen approprié à une source **25** capable de générer localement et au droit de l'intervalle entre molettes, une liaison entre les épaisseurs de substrat constitutives de la gaine **11.** La molette **23** porte à sa périphérie des empreintes positives **26** qui sont constituées par des barrettes axiales présentant, par exemple dans le plan median transversal, une encoche **27** dont la largeur correspond à la mesure des étranglements **8** à laisser subsister. Les barrettes **26** sont organisées pour successivement presser les deux épaisseurs de matière contre la molette **21,** de manière à générer localement une montée en température favorisant une liaison locale soit à partir des fibres, soit à partir de l'enduction d'un produit thermofusible. Cette thermofusion peut être provoquée par application de chaleur directe ou par l'intermédiaire d'ultrasons.

Bien que cela ne soit pas représenté, les molettes **21** et **23** peuvent être montées relativement pour que les barrettes **26** coopèrent avec la surface périphérique de la molette **21** avec ou sans pression d'application par exemple sous l'effet d'une contrainte élastique réglable.

Le duo de molettes **20** est entraîné en rotation synchrone dans le sens des flèches, de manière à provoquer le défilement dans le sens de la flèche **f**_{**1**} simultanément de la gaine **11** et de l'élément filaire **5** qui est maintenu dans un état d'étirement pour que sa section droite transversale se trouve être voisine par défaut de la largeur des encoches **27.** De la sorte, la rotation du duo de molettes à la vitesse convenable produit sur la gaine **11,** à plat et à l'état étendu, les zones de liaison **6** successivement espacées délimitant entre elles les sections **7** et ménageant chacune un étranglement **8.** Au-delà ou en aval du dispositif **20,** la contrainte d'étirement imposée à l'élément filaire cesse, de sorte que cet élément ré-adopte son état de repos entre deux étranglements successifs et produit le fronçage de la section **7** concernée.

Ce procédé de fabrication présente l'avantage, lors de la réalisation des étranglements **8,** de ne pas souder l'élément filaire à la gaine et de ne pas affecter la structure dudit élément filaire dont les caractéristiques mécaniques se trouvent ainsi préservées.

Il doit être considéré qu'un procédé de production en continu peut être mis en oeuvre pour une gaine tubulaire en tant qu'élément unitaire préalablement garni d'un élément filaire **5.**

De même, les dispositions ci-dessus sont applicables à un procédé faisant intervenir le garnissage de la coulisse avec au moins deux éléments filaires indépendants.

Les **fig. 5** et **6** montrent deux variantes de réalisation du duo de molettes **21.**

La **fig. 5** correspond à une coupe transversale de l'exemple selon la **fig. 4** dans laquelle seule la molette **23** est équipée des barrettes **26,** alors que la molette **21** présente un champ périphérique **28** lisse.

Dans l'exemple selon la **fig. 6,** les deux molettes **21** et **23** sont pourvues à leur périphérie des barrettes **26**_{**1**} et **26**_{**2**} qui sont exécutées pour délimiter entre elles et par les encoches **27**_{**1**} et **27**_{**2**} qu'elles ménagent, chacune la demi-section correspondant au passage de l'élément filaire **5.** Dans un tel cas, les molettes **21** et **23** sont alors entraînées exactement en vitesse synchrone, de manière à faire coïncider à chaque fois la mise en vis-à-vis des barrettes **26**_{**1**} et **26**_{**2**} en coïncidence contribuant à former les zones de liaison **6** et les immobilisations **8.**

La **fig. 7** montre une autre installation schématique pour la mise en oeuvre d'un procédé permettant de réaliser la coulisse conforme à l'invention. Une telle installation comprend un dispositif **20** qui est composé d'une sonotrode **30** tournant sur son axe **31** et dont la périphérie comporte, de place en place, des barrettes **32** destinées à réaliser les zones de liaison **8.** Le dispositif **20** comprend aussi une molette enclume **33** tournant sur son axe **34** et dont la périphérie présente une gorge continue **35** dont la largeur est légèrement inférieure à celle à l'état étiré de l'élément filaire **5.**

La gaine **11** est engagée entre la sonotrode **30** et la molette **33,** de manière à enrouler l'élément filaire **5** localement dans l'intervalle entre chants périphériques. De la sorte, l'élément filaire est correctement pris en charge et guidé par la gorge à l'intérieur de laquelle il est complètement engagé. Ainsi, lors de chaque passage d'une barrette **32,** l'élément filaire n'est pas affecté par l'opération de soudage haute fréquence qui forme les étranglements **8** et n'adhère pas à la gaine **11.**

### POSSIBILITÉ D'APPLICATION INDUSTRIELLE :

La coulisse de fronçage conforme à l'invention peut, par exemple, être mise en oeuvre sur des couches-culottes.

L'invention n'est pas limitée aux exemples décrits et représentés, car diverses modifications peuvent y être apportées sans sortir de son cadre.

## Revendications

1. Coulisse de fronçage du type comprenant :
- une gaine tubulaire **(1)** destinée à contenir au moins un élément filaire **(5)** étirable élastiquement,
- des zones de liaison **(6)** qui sont aménagées transversalement dans la gaine en étant espacées pour délimiter entre elles des sections tubulaires de fronçage **(7)** et qui réalisent des immobilisations locales (**8**) dudit élément filaire,
- au moins un élément filaire (**5**) disposé dans la gaine pour être retenu au droit des immobilisations et pour traverser librement et successivement les sections tubulaires en présentant localement dans chacune d'elle, d'une part, son état de repos **(5**_{**1**}**)** et, d'autre part, une longueur inférieure à celle de la section à laquelle il impose, par suite, un froncis d'orientation sensiblement perpendiculaire à la direction générale de la gaine,
**caractérisée en ce que** chaque zone de liaison (6) délimite un étranglement (8) présentant une section de passage sensiblement voisine de celle à l'état étiré **(5**_{**2**}**)** de l'élément filaire pour, d'une part, autoriser un déplacement relatif de l'élément filaire sous l'effet d'une contrainte dite de réglage et, d'autre part, immobiliser l'élément filaire lorsque ce dernier est au repos ou soumis à une contrainte inférieure à la contrainte de réglage.

2. Coulisse de fronçage selon la revendication 1, **caractérisée en ce que** l'élément filaire est constitué de plusieurs fils indépendants.

3. Coulisse de fronçage selon la revendication 1 ou 2, **caractérisée en ce que** les immobilisations **(8)** sont alignées pour définir un trajet rectiligne pour l'élément filaire.

4. Coulisse de fronçage selon la revendication 1 ou 2, **caractérisée en ce que** les immobilisations **(8)** définissent un trajet non rectiligne pour l'élément filaire.

5. Coulisse de fronçage selon l'une des revendications 1 à 4, **caractérisée en ce que** les immobilisations définissent au moins deux trajets pour au moins deux éléments filaires indépendants.

6. Coulisse selon l'une des revendications 1 à 5, **caractérisée en ce que** la gaine représente un fourreau plat indépendant.

7. Coulisse de fronçage selon l'une des revendications 1 à 5, **caractérisée en ce que** la gaine est formée par un rempli **(2)** d'une nappe fibreuse **(3).**

8. Coulisse de fronçage selon l'une des revendications 1 à 7, **caractérisée en ce que** les zones de liaison de la gaine sont formées par thermosoudage.

9. Article d'hygiène, tel qu'une couche culotte notamment, comprenant une partie froncée réalisée à partir d'une coulisse de fronçage selon l'une des revendications 1 à 8.

10. Procédé de réalisation d'une coulisse de fronçage selon l'une des revendications précédentes, **caractérisé en ce qu'**il consiste à :
- disposer d'une gaine mise à plat à l'état étendu,
- placer dans la gaine au moins un élément filaire étirable élastiquement qui est immobilisé localement et qui est soumis par ailleurs à une contrainte d'étirement longitudinal,
- réaliser localement dans la gaine, de façon espacée, des zones de liaison :
. qui délimitent dans la gaine des sections tubulaires de fronçage,
. et qui réalisent chacune une immobilisation constituée par un étranglement présentant une section de passage sensiblement voisine de celle de l'état étiré **(5**_{**2**}**)** de l'élément filaire et retenant ce dernier pour, d'une part, autoriser un déplacement relatif de l'élément filaire sous l'effet d'une contrainte dite de réglage et, d'autre part, immobiliser l'élément filaire lorsque ce dernier est au repos ou soumis à une contrainte inférieure à la contrainte de réglage,
- et à relâcher la contrainte d'étirement appliquée à l'élément filaire qui se rétracte à l'état de repos **(5**_{**1**}**)** entre les immobilisations et à l'intérieur de chaque section tubulaire à laquelle il impose un froncis.

11. Procédé selon la revendication 10, **caractérisé en ce qu'**on réalise les zones de liaison pour que les immobilisations définissent plusieurs alignements réservés à plusieurs éléments filaires.

12. Procédé selon l'une des revendications 10 ou 11, **caractérisé en ce qu'**on réalise la gaine par remplis d'un substrat en mettant à profit l'exécution des zones de liaison.

13. Procédé selon l'une des revendications 10 à 12, **caractérisé en ce qu'**il consiste à réaliser en continu la formation de la gaine, l'insertion de l'élément filaire dans la gaine, l'étirement longitudinal de l'élément filaire et l'exécution des zones de liaison.

14. Procédé selon l'une des revendications 10 à 13, **caractérisé en ce qu'**il consiste à réaliser les zones de liaison par thermosoudage.

15. Installation pour la mise en oeuvre du procédé et pour la réalisation d'une coulisse ou d'un article selon les revendications précédentes, **caractérisée en ce qu'**elle comprend :
- un dispositif de formation **(20)** composé d'un duo de molettes tournantes superposées comprenant :
. une molette enclume **(21, 33),**
. une molette de soudage **(23, 30),**
- des moyens pour faire passer une nappe et une gaine **(11)** entre lesdites molettes avec insertion d'au moins un élément filaire étirable élastiquement,
- des moyens de mise sous tension **(13)** dudit élément filaire,
- et des barrettes **(26, 32)** portées par l'une des molettes pour exécuter sur la gaine des zones de liaison **(6)** définissant un étranglement présentant une section de passage sensiblement voisine de celle de l'état étiré de l'élément filaire.

16. Installation selon la revendication 15, **caractérisée en ce que** l'une des deux molettes délimite une sorte de gorge périphérique réservée au passage de l'élément filaire.

17. Installation selon la revendication 16, **caractérisée en ce que** la molette de soudage est une sonotrode, **en ce qu'**elle porte à sa périphérie des barrettes **(32)** et **en ce que** la molette enclume comporte, à sa périphérie, une gorge **(35)** dont la largeur est légèrement inférieure à celle à l'état étiré de l'élément filaire.

## Patentansprüche

1. Geraffter Tunnelsaum mit
- einer röhrenförmigen Hülle (1), die wenigstens ein elastisch dehnbares, fadenförmiges Element (5) enthält,
- Verbindungsbereichen (6), die in der Hülle quer angeordnet und zueinander beabstandet sind, um dazwischen röhrenförmige geraffte Abschnitte (7) zu begrenzen, und die örtliche Halterungen (8) des fadenförmigen Elements herstellen,
- wenigstens einem fadenförmigen Element (5), das in der Hülle angeordnet ist, um rechtwinklig zu den Halterungen gehalten zu werden und um die röhrenförmigen Abschnitte nacheinander ungehindert zu durchqueren, wobei es örtlich in jedem der Abschnitte einerseits einen Ruhezustand (5₁) und andererseits eine Länge aufweist, die kürzer ist als der Abschnitt, der dadurch gerafft wird, und zwar in einer im wesentlichen quer zur Hauptrichtung der Hülle verlaufenden Richtung,
**dadurch gekennzeichnet, daß** jeder Verbindungsbereich (6) eine Verengung (8) begrenzt, die einen Durchgangsquerschnitt aufweist, der nahezu dem Querschnitt des fadenförmigen Elements in gedehntem Zustand (5₂) entspricht, um einerseits eine relative Verschiebung des fadenförmigen Elements unter der Einwirkung einer sogenannten Regulierungsspannung zu ermöglichen und andererseits das fadenförmige Element zu halten, wenn sich dieses im Ruhezustand befindet oder einer Spannung ausgesetzt ist, die kleiner ist als die Regulierungsspannung.

2. Geraffter Tunnelsaum nach Anspruch 1,
**dadurch gekennzeichnet, daß** das fadenförmige Element aus verschiedenen separaten Fäden besteht.

3. Geraffter Tunnelsaum nach Anspruch 1 oder 2,
**dadurch gekennzeichnet, daß** die Halterungen (8) in einer Reihe ausgerichtet sind, um einen geradlinigen Verlauf des fadenförmigen Elements zu bestimmen.

4. Geraffter Tunnelsaum nach Anspruch 1 oder 2,
**dadurch gekennzeichnet, daß** die Halterungen (8) einen nicht geradlinigen Verlauf des fadenförmigen Elements bestimmen.

5. Geraffter Tunnelsaum nach einem der Ansprüche 1 bis 4,
**dadurch gekennzeichnet, daß** die Halterungen wenigstens zwei Bahnen für wenigstens zwei voneinander unabhängige fadenförmige Elemente bestimmen.

6. Tunnelsaum nach einem der Ansprüche 1 bis 5,
**dadurch gekennzeichnet, daß** die Hülle ein separates flaches Futteral darstellt.

7. Geraffter Tunnelsaum nach einem der Ansprüche 1 bis 5,
**dadurch gekennzeichnet, daß** die Hülle durch einen Einschlag (2) einer Faserschicht (3) gebildet wird.

8. Geraffter Tunnelsaum nach einem der Ansprüche 1 bis 7,
**dadurch gekennzeichnet, daß** die Verbindungsbereiche der Hülle durch Heißversiegeln gebildet werden.

9. Hygieneartikel, wie zum Beispiel insbesondere ein Windelhöschen, mit einem gerafften Teil, das ausgehend von einem gerafften Tunnelsaum nach einem der Ansprüche 1 bis 8 ausgeführt ist.

10. Verfahren zur Herstellung eines gerafften Tunnelsaums nach einem der vorhergehenden Ansprüche,
**dadurch gekennzeichnet, daß** es folgendes umfaßt:
- eine in ausgebreitetem Zustand flach liegende Hülle,
- Einlegen wenigstens eines elastisch dehnbaren, fadenförmigen **Elements** in die Hülle, welches örtlich gehalten wird und außerdem einer längsgerichteten Dehnungsspannung ausgesetzt ist,
- Herstellen von voneinander beabstandeten örtlichen Verbindungsbereichen in der Hülle, welche
. in der Hülle röhrenförmige, geraffte Abschnitte begrenzen,
. und jeweils eine Halterung herstellen, die durch eine Verengung gebildet wird, die einen Durchgangsquerschnitt aufweist, der nahezu dem Querschnitt des fadenförmigen Elements in gedehntem Zustand (5₂) entspricht, und die dieses hält, um einerseits eine relative Verschiebung des fadenförmigen Elements unter Einwirkung einer sogenannten Regulierungsspannung zu ermöglichen und andererseits das fadenförmige Element zu halten, wenn sich dieses im Ruhezustand befindet oder einer Spannung ausgesetzt ist, die kleiner ist als die Regulierungsspannung,
- Lockern der auf das fadenförmige Element ausgeübten Dehnungsspannung, welches sich im Ruhezustand (5₁) zwischen den Halterungen im Inneren eines jeden gerafften röhrenförmigen Abschnitts zusammenzieht.

11. Verfahren nach Anspruch 10,
**dadurch gekennzeichnet, daß** Verbindungsbereiche ausgeführt werden, um Halterungen in mehreren Reihen zu bilden, die für mehrere fadenförmige Elemente vorgesehen sind.

12. Verfahren nach einem der Ansprüche 10 oder 11,
**dadurch gekennzeichnet, daß** die Hülle durch Einschlagen eines Ausgangsmaterials hergestellt wird, wobei die Ausführung der Verbindungsbereiche genutzt wird.

13. Verfahren nach einem der Ansprüche 10 bis 12,
**dadurch gekennzeichnet, daß** es darin besteht, die Bildung der Hülle, das Einfügen des fadenförmigen Elements in die Hülle, das Dehnen des fadenförmigen Elements in Längsrichtung und die Herstellung der Verbindungsbereiche im Endlosverfahren auszuführen.

14. Verfahren nach einem der Ansprüche 10 oder 13,
**dadurch gekennzeichnet, daß** es darin besteht, die Verbindungsbereiche durch Heißsiegeln auszuführen.

15. Anlage zur Ausführung des Verfahrens und zur Herstellung eines Tunnelsaums oder eines Artikels nach den vorhergehenden Ansprüchen,
**dadurch gekennzeichnet, daß** sie folgendes umfaßt:
- eine Vorrichtung zur Bildung eines Tunnelsaums (20) bestehend aus zwei übereinanderliegenden, sich drehenden Rollen mit
. einer Auflagerolle (21, 33),
. einer Rollenelektrode (23, 30),
- Mittel zum Durchführen einer Faserschicht und einer Hülle (11) zwischen diesen Rollen und Einlegen wenigstens eines elastisch dehnbaren fadenförmigen Elements,
- Mittel zum Spannen (13) des fadenförmigen Elements,
- und Stege (26, 32) an einer der Rollen, um auf der Hülle Verbindungsbereiche (6) auszuführen, die eine Verengung bilden, welche einen Durchgangsquerschnitt aufweist, der nahezu dem des fadenförmigen Elements in gedehntem Zustand entspricht.

16. Anlage nach Anspruch 15,
**dadurch gekennzeichnet, daß** eine der beiden Rollen eine Art umlaufende Nut begrenzt, die für den Durchzug des fadenförmigen Elements vorgesehen ist.

17. Anlage nach Anspruch 16,
**dadurch gekennzeichnet, daß** die Rollenelektrode eine Sonotrode ist, daß sie an ihrem Umfang mit Stegen (32) versehen ist und daß die Auflagerolle an ihrem Umfang eine Nut (35) aufweist, deren Breite etwas kleiner ist als die des fadenförmigen Elements in gedehntem Zustand.

## Claims

1. Ruffling slide of the type comprising:
- a tubular sleeve (1) intended to contain at least one elastically stretchable wire-like element (5),
- connecting portions (6) which are arranged transversely in the sleeve, being spaced apart to define therebetween tubular ruffling portions (7) and which effect local immobilisations (8) of said wire-like element, and
- at least one wire-like element (5) disposed in the sleeve to be retained at the level of the immobilisations and to traverse freely and successively the tubular portions, presenting locally in each of them, on the one hand, its inoperative position (5₁) and, on the other hand, a length less than that of the portion on which it subsequently imposes a ruffling of orientation substantially perpendicular to the general direction of the sleeve,
**characterised in that** each connecting portion (6) defines a narrowed portion (8) presenting a section of passage substantially close to that of the wire-like element in the stretched state (5₂), in order, on the one hand, to allow a relative displacement of the wire-like element under the effect of a so-called adjusting stress and, on the other hand, to immobilise the wire-like element when the latter is in the inoperative position or subjected to a stress less than the adjusting stress.

2. Ruffling slide according to claim 1, **characterised in that** the wire-like element is constituted by a plurality of independent wires.

3. Ruffling slide according to claim 1 or 2, **characterised in that** the immobilisations (8) are aligned to define a rectilinear path for the wire-like element.

4. Ruffling slide according to claim 1 or 2, **characterised in that** the immobilisations (8) define a non-rectilinear path for the wire-like element.

5. Ruffling slide according to one of claims 1 to 4, **characterised in that** the immobilisations define at least two paths for at least two independent wire-like elements.

6. Ruffling slide according to one of claims 1 to 5, **characterised in that** the sleeve represents an independent flat sheath.

7. Ruffling slide according to one of claims 1 to 5, **characterised in that** the sheath is formed by a pleat (2) of a fibrous lap (3).

8. Ruffling slide according to one of claims 1 to 7, **characterised in that** the connecting portions of the sleeve are formed by heat-sealing.

9. Sanitary article, such as a diaper or a nappy for example, comprising a ruffling part made from a ruffling slide according to one of claims 1 to 8.

10. Method for producing a ruffling slide according to one of the preceding claims, **characterised in that** in consists in:
- disposing a sleeve flat in the extended state,
- placing in the sleeve at least one elastically stretchable wire-like element which is immobilised locally and which is subjected, furthermore, to a longitudinal stretching stress,
- locally making in the sleeve, in spaced apart manner, connecting portions:
. which define in the sleeve tubular ruffling portions,
. and which each effect an immobilisation constituted by a narrowed portion presenting a section of passage substantially close to that of the stretched state (5₂) of the wire-like element and retaining this latter, in order, on the one hand, to allow a relative displacement of the wire-like element under the effect of a so-called adjusting stress and, on the other hand, to immobilise the wire-like element when the latter is in inoperative position or subjected to a stress less than the adjusting stress,
- and releasing the stretching stress applied to the wire-like element which contracts to the inoperative position (5₁) between the immobilisations and inside each tubular portion on which it imposes a ruffle.

11. Method according to claim 10, **characterised in that** the connecting portions are made so that the immobilisations define a plurality of alignments reserved for a plurality of wire-like elements.

12. Method according to claim 10 or 11, **characterised in that** the sleeve is made by folding a substrate, taking advantage of the making of the connecting portions.

13. Method according to one of claims 10 to 12, **characterised in that** it consists in continuously effecting formation of the sleeve, the insertion of the wire-like element in the sleeve, the longitudinal stretching of the wire-like element and the making of the connecting portions.

14. Method according to one of claims 10 to 13, **characterised in that** it consists in making the connecting portions by heat-sealing.

15. Installation for carrying out the method and for making a slide or an article according to the preceding Claims, **characterised in that** it comprises:
- a forming device (20) composed of a duo of superposed rotating wheels comprising:
. an anvil wheel (21, 33),
. a welding wheel (23, 30),
- means for passing a lap and a sleeve (11) between said wheels with insertion of at least one elastically stretchable wire-like element,
- means (13) for tensioning said wire-like element, and
- bars (26, 32) borne by one of the wheels to make on the sleeve connecting portions (6) defining a narrowed portion presenting a section of passage substantially close to that of the stretched state of the wire-like element.

16. Installation according to claim 15, **characterised in that** one of the two wheels defines a sort of peripheral groove reserved for the passage of the wire-like element.

17. Installation according to claim 16, **characterised in that** the welding wheel is a sonotrode, **in that** it bears bars (32) on its periphery, and **in that** the anvil wheel comprises on its periphery a groove (35) whose width is slightly smaller than that of the wire-like element in the stretched state.
